Europäisches Patentamt

European Patent Office

Office européen des brevets

(19)

(11) Publication number:

**0 043 675**
**B1**

(12) **EUROPEAN PATENT SPECIFICATION**

(45) Date of publication of patent specification: **03.10.84**

(51) Int. Cl.³: **C 07 G 7/00, A 61 K 37/14**

(21) Application number: **81302858.6**

(22) Date of filing: **25.06.81**

(54) Modified hemoglobins suitable for use as oxygen carriers for blood substitutes.

(30) Priority: **02.07.80 JP 90316/80**

(43) Date of publication of application:
**13.01.82 Bulletin 82/02**

(45) Publication of the grant of the patent:
**03.10.84 Bulletin 84/40**

(84) Designated Contracting States:
**CH DE FR GB LI SE**

(56) References cited:
**DE-A-2 616 086**
**US-A-4 064 118**

(73) Proprietor: **AJINOMOTO CO., INC.**
**5-8, Kyobashi 1-chome, Chuo-ku**
**Tokyo 104 (JP)**

(72) Inventor: **Ajisaka, Katsumi**
**1293-4, Nakano-cho Totsuka-ku**
**Yokohama-shi Kanagawa-ken (JP)**
Inventor: **Iwashita, Yuji**
**1212 Ichinotsubo-Apartment 581-1 Ichinotsubo**
**Nakahara-ku Kawasaki-shi Kanagawa-ken (JP)**

(74) Representative: **Jones, Michael Raymond et al**
**HASELTINE LAKE & CO. 28 Southampton**
**Buildings Chancery Lane**
**London WC2A 1AT (GB)**

The file contains technical information
submitted after the application was filed and
not included in this specification

Courier Press, Leamington Spa, England.

**Description**

This invention relates to modified hemoglobins suitable for use as oxygen carriers for blood substitutes.

US—A—4064118 discloses a blood substitute or blood extender prepared by chemically coupling hemoglobin with a polysaccharide material selected from dextran and hydroxyethyl starch, and having a molecular weight of from about 5,000 to about 2,000,000, to form a covalently bonded chemical complex. The complex has similar oxygen transporting abilities to hemoglobin, and has a much lower rate of renal excretion.

According to the present invention, there is provided a combined hemoglobin or derivative thereof, to which inulin is covalently bound, there being from 1 to 15 molecules of inulin bound to each hemoglobin sub-unit.

The basis of the present invention is that newly prepared hemoglobins modified by combination with inulin have the following excellent properties for use in a blood substitute:—

(1) when the modified hemoglobin is infused, the residence time in the blood circulation is long enough to serve effectively as an oxygen carrier of a blood substitute;
(2) the modified hemoglobin has the ability to supply oxygen to tissues;
(3) the modified hemoglobin is easily metabolised and does not accumulate in the body;
(4) the modified hemoglobin is a nontoxic material; and
(5) the modified hemoglobin not only carries oxygen but also works as a plasma expander.

The products of the present invention are hemoglobin derivatives of inulin, which are very different from the hemoglobin products combined with HES or dextran found in the prior art; moreover, the products of the present invention have very excellent properties as a blood substitute, compared with those of hemoglobin products combined with HES or dextran.

The hemoglobin used in the present invention includes natural hemoglobins such as from a human being, or from animals such as cow, pig, sheep, horse, dog, monkey, or hen. The hemoglobin used in the present invention may also be a hemoglobin derivative, e.g. a sugar nucleotide, alcohol, carboxylic acid, sulphate such as pyridoxal-5'-sulphate, or phosphate such as pyridoxal-5'-phosphate derivative.

The inulin which is combined with the hemoglobin of the present invention is preferably in the range of 4,000 to 6,000 daltons of molecular weight and it may have branches in its molecule.

The hemoglobin may be combined with the inulin by any suitable process. For instance, the amino group of the hemoglobin can be coupled to the hydroxy of the inulin by a coupling agent such as cyanogen bromide, or by a polyfunctional crosslinking agent such as cyanuric chloride, 2,2'-dichlorobenzidine, p,p'-difluoro-m,m'-dinitrodiphenylsulphone or 2,4-dichloronitrobenzene.

Furthermore inulin can be combined with hemoglobin using a dicarboxylic acid such as succinic acid, glutaric acid, or adipic acid. Here, inulin is esterified with one of the carboxy groups of one of these dicarboxylic acids or an anhydride thereof, and then the free carboxy group of the dicarboxylic acid monoester is combined with hemoglobin by activating that group with a conventional carboxy group-activating agent, such as N-hydroxy succinimide, pentachlorophenol, imidazole, succinimide or carbodiimidazole.

In the case that a hemoglobin derivative is used as a starting material, the same general reaction procedure can be applied.

For example, pyridoxal phosphate may first be coupled to hemoglobin by the known method described by R. E. Benesch, R. Benesch, R. D. Renthal, and N. Maeda in Biochemistry, *11*, 3675 (1972). Then, as a next step, inulin can be combined by the procedure described above.

Although in theory, with one sub-unit of hemoglobin, from 1 to 20 molecules of inulin could be coupled, nevertheless, it is important, from the point of view of serving as an oxygen carrier for a blood substitute, that the viscosity of the solution of the oxygen carrier is not too high. From this point of view, the number of molecules of inulin attached to each hemoglobin sub-unit needs to be from 1 to 15.

The present invention will be further illustrated by the following Examples.

Example 1

Inulin (10 g, 0.002 mole, produced by Tokyo Kasei Co., of Tokyo, Japan) was dissolved in 100 ml water and to this solution cyanogen bromide (1 g, 0.01 mole) in solution in dioxane (10 ml) was added dropwise at ice temperature. During the reaction, the pH of the resulting mixture was kept in the range from 9 to 10 by the addition of 2*N* NaOH solution.

The addition of cyanogen bromide was completed after 30 minutes and then the mixture was stirred for a further period of 20 minutes.

The resulting reaction mixture was poured into cold acetone (400 ml). The activated inulin was obtained as a suspension which was allowed to stand for several minutes, after which the supernatant was removed by decantation. The unreacted cyanogen bromide was washed out with cold acetone (300 ml). After the complete removal of the remaining acetone, an aqueous solution (50 ml) containing stroma-free hemoglobin (5.2 g, 0.08 mmole) was added. The resulting mixture was then stirred for 4 hours at 4°C.

The mixture was ultrafiltered and concentrated by an "XM-100" membrane (obtained from Amicon Co., U.S.A.).

To see the relationship between the residence time in the circulation and the molecular weight of the modified hemoglobin of the present invention, a fraction of higher molecular weight and a fraction of lower molecular weight of the hemoglobin covalently modified by inulin were separated into approximately equal amounts by gel permeation chromatography using "Sephadex G 100" (available from Pharmacea Co., Sweden). Hereafter, these two fractions are called "Inulin-Hb 1" and "Inulin-Hb 2", respectively.

The number of inulin molecules which were combined with one sub-unit of hemoglobin, was determined as follows:

The solution of inulin-combined hemoglobin, of which the volume was $V_0$ and the concentration as hemoglobin was $C_0$ (as determined by the cyanomethemoglobin method), was lyophilized and its dry weight was measured. The number (n) of inulin molecules, which are combined with one hemoglobin sub-unit, was obtained by the following equation:—

$$n = \frac{(m_o - C_o V_o)/M_I}{C_o V_o / M_H}$$

In this equation, $M_I$ is the average molecular weight of the inulin used, $M_H$ is the molecular weight of hemoglobin subunit, and $m_o$ is the net weight of the inulin-combined hemoglobin after lyophilization.

Therefore the molecular weight (M) of an inulin-combined hemoglobin sub-unit can be expressed by the following equation:—

$$M = M_H + nM_I$$

The number of the combined inulin molecules and the molecular weight, M, of the substance obtained by this method are shown in the following Table 1.

TABLE 1

| Substance | Number of combined inulin molecules per hemoglobin sub-unit | Molecular weight of combined substance incorporating one sub-unit |
|---|---|---|
| Inulin-Hb 1 | 1.7 | $2.5 \times 10^4$ |
| Inulin-Hb 2 | 6.8 | $5.1 \times 10^4$ |

Example 2

To a 100 ml solution of inulin (5 g, 0.001 mole), there was added dropwise cyanuric chloride (1 g, 0.005 mole) in a mixture of 10 ml water and 20 ml acetone. During the reaction, the pH of the reaction mixture was kept between 10 and 11 by the addition of 2N NaOH solution.

The mixture was stirred for 5 minutes after completing the addition of cyanuric chloride, and then the pH of it was adjusted to 3 by 2N HCl solution. The reaction mixture was poured into 500 ml acetone and the precipitate which resulted was collected by filtration and washed with acetone, and dried. The obtained product was dissolved in 100 ml 0.2 M borate buffer and then 5.6% stroma-free hemoglobin solution (15 ml) was added. The resulting mixture was stirred for 3 hours in an ice bath. The reaction mixture was treated by the same general procedure as described in Example 1. Two fractions "Inulin-Hb 3" and "Inulin-Hb 4" were prepared by gel permeation chromatography as in Example 1.

The number of the inulin molecules in these inulin-combined hemoglobins was determined in the same time method as in Example 1, and that number and the estimated average molecular weight are given in the following Table 2.

TABLE 2

| Substance | Number of combined inulin molecules per hemoglobin sub-unit | Molecular weight of combined substance incorporating one sub-unit |
|---|---|---|
| Inulin-Hb 3 | 3.9 | $3.6 \times 10^4$ |
| Inulin-Hb 4 | 7.4 | $5.4 \times 10^4$ |

Example 3

Instead of the stroma-free hemoglobin solution as was used in Example 2, 6.3% aqueous solution of pyridoxylated hemoglobin (13.3 ml) was employed and the procedure described in Example 2 was repeated. "Inulin-Hb 5" was obtained by concentration of the reaction mixture through a membrane "XM-100" (produced by Amicon Co., USA).

The number of the combined inulin molecules and the molecular weight of the "Inulin-Hb 5" were 4.2 and $3.7 \times 10^4$, respectively, per one sub-unit of hemoglobin.

Example 4

Inulin (5.4 g, 0.03 mole) and succinic anhydride (3.0 g, 0.03 mole) were dissolved in pyridine (50 ml) and the resulting solution was refluxed for two hours. Then $n$-hexane (200 ml) was added to the solution and the precipitate which resulted was filtered and washed with $n$-hexane. The precipitate (2.8 g), N-hydroxysuccinimide (2.4 g, 0.021 mole), and dicyclohexylcarbodiimide (4.2 g, 0.021 mole) were dissolved in 60 ml N,N-dimethylformamide and the reaction mixture solution was refluxed for one hour. After filtering off the dicyclohexylurea, $n$-hexane (300 ml) was added to the filtrate.

The precipitate of activated inulin was filtered and washed with $n$-hexane. The activated inulin (2.1 g, 0.0004 mole) was added to a 1% hemoglobin solution (100 ml, 0.000016 mole) of 0.2 $M$ borate buffer (pH 8.5). The reaction mixture was stirred for 16 hours at 4°C, ultrafiltered, and concentrated using a "PM-30" membrane (produced by Amicon Co., USA).

The resulting product, "Inulin-Hb 6", incorporated three inulin molecules and its molecular weight was $3.1 \times 10^4$, per one sub-unit of hemoglobin.

Example 5

A one percent solution of pyridoxylated hemoglobin in 0.2 $M$ borate buffer (100 ml, 0.000016 mole) instead of the unmodified hemoglobin used in Example 4, was used for the reaction with the activated inulin, following the same general procedure as in Example 4. The obtained substance, "Inulin-Hb 7", incorporated 2.5 inulin molecules and the molecular weight was $2.9 \times 10^4$, per one sub-unit of hemoglobin.

Example 6

The half residence time (T-50) in the circulation of rats was determined as follows; male Spaque Dawley rats (200—300 g) were anaesthetized by the ip injection of 400 mg/kg pentobarbital. After the phlebotomy of 5 ml/kg blood through a polyethylene catheter inserted in the femoral vein the same volume of a hemoglobin or inulin-combined hemoglobin solution (concentration 4—6%) was infused through the same catheter.

Aliquots (0.5 ml) of the blood were collected at 5, 10, 30, 60, 90 and 120 minutes after the infusion and then the concentration of the injected derivatives in the plasma was measured by the cyanomethemoglobin method after centrifuging. The half residence time was obtained by semilogarithmic plots of the concentration against the time after the infusion.

The results obtained are given in the following Table 3.

TABLE 3

| Substance | Half residence time (minutes) | $P_{50}$ value* (Pa) |
|---|---|---|
| Inulin-Hb 1 | 90 | 272 (2.05 mm Hg) |
| Inulin-Hb 2 | 110 | 195 (1.46 mm Hg) |
| Inulin-Hb 3 | 110 | 126 (0.94 mm Hg) |
| Inulin-Hb 4 | 120 | 96 (0.72 mm Hg) |
| Inulin-Hb 5 | 100 | 167 (1.25 mm Hg) |
| Inulin-Hb 6 | 90 | 513 (3.85 mm Hg) |
| Inulin-Hb 7 | 80 | 587 (4.40 mm Hg) |
| Hemoglobin (control) | 40 | 880 (6.60 mm Hg) |
| Dextran-Hb (control) | 70 | 160 (1.20 mm Hg) |

*25°C, 0.1 $M$ Na Cl solution, pH 7.4.

4

**0 043 675**

The oxygen dissociation curves of these inulin-combined hemoglobins were measured by the apparatus, which was made by Dr. Imai (as described by K. Imai, H. Morimoto, M. Kotani, H. Watari, H. Waka, and M. Kuroda, in Biochem. Biophys. Acta *200*, 189—196 (1970)).

The values of $P_{50}$, at which pressure half of the oxygen coordinated to hemoglobin substance was released, were estimated from the dissociation curves and are given in the foregoing Table 3.

Dextran-hemoglobin (molecular weight: over $2.0 \times 10^5$) quoted in Table 3 as a control (reference) substance was prepared by combining dextran (molecular weight: $1.6 \times 10^5$) with hemoglobin by the help of cyanuric chloride.

As indicated from these results, the half residence time in the circulation of the inulin-combined hemoglobins is much longer than that of hemoglobin itself and these substances have the ability to supply sufficient oxygen to the tissues, too.

Furthermore, these inulin-combined hemoglobins are good plasma expanders. Therefore, these substances are very useful in blood substitutes.

## Claims

1. A combined hemoglobin or derivative thereof, to which inulin is covalently bound, there being from 1 to 15 molecules of inulin bound to each hemoglobin sub-unit.

2. A combined hemoglobin or derivative thereof, according to Claim 1, wherein the hemoglobin is that from a human being or from a cow, pig, sheep, horse, dog, monkey or hen.

3. A combined hemoglobin or derivative thereof, according to Claim 1 or 2, wherein the hemoglobin derivative is a sugar nucleotide, alcohol, carboxylic acid, sulphate or phosphate derivative.

4. A combined hemoglobin or derivative thereof according to any preceding claim, wherein the inulin has a molecular weight in the range from 4000 to 6000 daltons.

5. A blood substitute which includes as an oxygen carrier a combined hemoglobin or derivative thereof according to any preceding claim.

6. A process for producing a combined hemoglobin or derivative thereof according to any preceding claim, wherein the amino group of the hemoglobin or derivative thereof is coupled to the hydroxy group of the inulin by a coupling agent or crosslinking agent.

7. A process according to Claim 6, wherein the agent is cyanogen bromide, cyanuric chloride, 2,2-dichlorobenzidine, p,p'-difluoro-m,m'-dinitrodiphenylsulphone, or 2,4-dichloronitrobenzene.

8. A process for producing a combined hemoglobin or derivative thereof according to any one of Claims 1 to 5, wherein inulin is esterified with a dicarboxylic acid or anhydride thereof, and then the free carboxy group of the dicarboxylic acid monoester is combined with hemoglobin by activating that group with a conventional carboxy group-activating agent.

9. A combined hemoglobin or derivative thereof, according to any one of Claims 1 to 4, wherein the hemoglobin derivative is a pyridoxal-5'-phosphate or pyridoxal-5'-sulphate derivative.

## Patentansprüche

1. Gebundenes Hämoglobin oder dessen Derivat, an das Inulin covalent gebunden ist, wobei 1 bis 15 Inulin-Moleküle an jede Hämoglobin-Untereinheit gebunden sind.

2. Gebundenes Hämoglobin oder dessen Derivat gemäß Anspruch 1, wobei das Hämoglobin menschliches Hämoglobin ist oder vom Rind, Schwein, Schaf, Pferd, Hund, Affen oder Huhn stammt.

3. Gebundenes Hämoglobin oder dessen Derivat gemäß Anspruch 1 oder 2, wobei als Hämoglobin-Derivat ein Zucker-nucleotid-, Alkohol-, Carbonsäure-, Sulfat- oder Phosphat-Derivat vorliegt.

4. Gebundenes Hämoglobin oder dessen Derivat gemäß einem der vorhergehenden Ansprüche, worin das Inulin ein Molekulargewicht im Bereich von 4000 bis 6000 Dalton hat.

5. Blutersatzmittel, enthaltend als Sauerstoffträger ein gebundenes Hämoglobin oder dessen Derivat gemäß einem der vorhergehenden Ansprüche.

6. Verfahren zur Herstellung eines gebundenen Hämoglobins oder dessen Derivat gemäß einem der vorhergehenden Ansprüche, bei dem die Aminogruppe des Hämoglobins oder dessen Derivat mit Hilfe eines Kupplungsmittels oder Vernetzungsmittels mit der Hydroxylgruppe des Inulins gekuppelt wird.

7. Verfahren gemäß Anspruch 6, bei dem das Mittel Bromcyan, Cyanurchlorid, 2,2-Dichlorbenzidin, p,p'-Difluor-m,m'-dinitrodiphenylsulfon oder 2,4-Dichlornitrobenzol ist.

8. Verfahren zur Herstellung eines gebundenen Hämoglobins oder dessen Derivat gemäß einem der Ansprüche 1 bis 5, bei dem Inulin mit einer Dicarbonsäure oder deren Anhydrid verestert und dann die freie Carboxylgruppe des Dicarbonsäure-monoesters durch Aktivierung dieser Gruppe mit Hilfe eines üblichen Carboxylgruppen-Aktivierungsmittels an das Hämoglobin gebunden wird.

9. Gebundenes Hämogloibin oder dessen Derivat gemäß einem der Ansprüche 1 bis 4, worin das Hämoglobin-Derivat ein Pyridoxal-5'-phosphat- oder Pyridoxal-5'-sulfat-Derivat ist.

**0 043 675**

## Revendications

1. Une hémoglobine (ou un de ses dérivés) combinée, à laquelle de l'inuline est unie par covalence, dans laquelle il y a 1 à 15 molécules d'inuline fixées à chaque sous-unité d'hémoglobine.

2. Une hémoglobine (ou un de ses dérivés) combinée selon la revendication 1, dans laquelle l'hémoglobine est celle d'un être humain ou d'une vache, d'un porc, d'un mouton, d'un cheval, d'un chien, d'un singe ou d'une poule.

3. Une hémoglobine (ou un de ses dérivés) combinée selon la revendication 1 ou 2, dans laquelle le dérivé d'hémoglobine est un dérivé de sucre-nucléotide, d'alcool, d'acide carboxylique, de sulfate ou de phosphate.

4. Une hémoglobine (ou un de ses dérivés) combinée selon l'une quelconque des revendications précédentes, dans laquelle l'inuline a un poids moléculaire dans la gamme de 4 000 à 6 000 daltons.

5. Un succédané du sang qui comprend comme véhicule d'oxygène une hémoglobine (ou un de ses dérivés) combinée selon l'une quelconque des revendications précédentes.

6. Un procédé pour la production d'hémoglobine (ou d'un de ses dérivés) combinée selon l'une quelconque des revendications précédentes, dans lequel le groupe amino de l'hémoglobine ou d'un de ses dérivés est couplé ou groupe hydroxy de l'inuline par un agent de couplage ou un agent de réticulation.

7. Un procédé selon la revendication 6, dans lequel l'agent est le bromure de cyanogène, le chlorure cyanurique, la 2,2-dichlorobenzidine, la p,p'-difluoro-m,m'-dinitrodiphénylsulfone ou le 2,4-dichloronitrobenzène.

8. Un procédé pour la production d'une hémoglobine (ou d'un de ses dérivés) combinée selon l'une quelconque des revendications 1 à 5, dans lequel de l'inuline est estérifiée avec un acide ou un anhydride dicarboxylique, puis le groupe carboxy libre du monoester d'acide dicarboxylique est combiné avec l'hémoglobine par activation de ce groupe avec un agent classique d'activation d'un groupe carboxy.

9. Une hémoglobine (ou un de ses dérivés) combinée selon l'une quelconque des revendications 1 à 4, dans laquelle le dérivé d'hémoglobine est un dérivé de pyridoxal-5'-phosphate ou de pyridoxal-5'-sulfate.

6